# EUROPEAN PATENT APPLICATION

(11) **EP 3 025 751 A1**
(43) Date of publication of application: **01.06.2016**
(21) Application number: 14829043.0
(22) Date of filing: 10.07.2014
(51) Int. Cl.: A61M 25/00, A61B 1/00, A61N 5/067

(54) **MEDICAL DEVICE AND LIGHT-EMITTING PROBE MOUNTING KIT FOR MEDICAL DEVICE**

(30) Priority: 26.07.2013 JP 2013155867
(71) Applicant: Arai Medphoton Research Laboratories, Corporation, Kawasaki-shi, Kanagawa 211-0063 (JP)
(72) Inventor: ARAI, Tsunenori, Kawasaki-shi Kanagawa 211-0063 (JP); ITO, Arisa, Kawasaki-shi Kanagawa 211-0063 (JP); OGAWA, Emiyu, Kawasaki-shi Kanagawa 211-0063 (JP)
(74) Representative: Grünecker Patent- und Rechtsanwälte PartG mbB
(86) International application number: PCT/JP2014/068410
(87) International publication number: WO 2015/012116

(57) **Abstract**

A light-emitting probe mounting kit that can easily be combined with and externally mounted to a medical device that is selected by an operator. The light-emitting probe mounting kit for a medical device is provided with: a light transmission section (22) that transmits light from a light source; a light-emitting probe (21) that is connected to the light transmission section (22) and that emits the light that is transmitted by the light transmission section (22); and externally mounting sections (31, 32) that are for externally mounting the light-emitting probe (21) to an outer portion of a medical device (10) that is inserted and used within a living body. The externally mounting sections (31, 32) comprise a heat-shrinkable tube having a space (S) into which the medical device (10) can be inserted prior to heat shrinkage. The heat-shrinkable tubes (31, 32) are fixed to the light-emitting probe (21).

## Description

### TECHNICAL FIELD

The present invention relates to a medical device with a light-emitting probe mounted to the outer side thereof, and a kit for mounting a light-emitting probe for a medical device.

### BACKGROUND ART

As a treatment method of arrhythmias, a radiofrequency catheter ablation is generally performed. The radiofrequency catheter ablation is a method of treating a drug-resistant tachyarrhythmia or an extrasystole, by adding a thermal energy made by a high frequency to an origin of occurrence or a reentry circuit of an arrhythmias from a tip of a catheter.

In the radiofrequency catheter ablation, an operator carries out an electrical conduction interruption with a catheter which is different from a catheter for carrying out an electrode block, while checking an electric block with an electrode catheter.

In a treatment of an arrhythmia, it is difficult to isolate the focus thereof, and therefore, it is necessary to set a line for cutting off an abnormal electrical signal. When two pulmonary veins are isolated together, a good result is obtained.

However, since a radiofrequency catheter ablation sends an electricity to points one by one, it is necessary to connect the points in order to obtain a linear region for treatment at a time, and thus, it is difficult to obtain a continuous region for treatment.

Meanwhile, in recent years, researches have made progress in photodynamic therapy: PDT, also referred to as photochemical treatment, as a method of treating cancers or arrhythmia.

The photodynamic therapy is a method of treatment, in which a photosensitive substance is administered by a method such as intravenous injection, and a target tissue in a state of having the photosensitive substance distributed therein is irradiated with a beam such as a laser beam to cause a photosensitized reaction induced by the photosensitive substance and the light, and oxygen, and cells in the target tissues are necrosed by this photosensitized reaction.

Known as a laser treatment system to perform the photodynamic therapy are those provided with a laser catheter and a main body of a PDT apparatus, in which the main body of a PDT apparatus emits a laser beam to the laser catheter and detects a return light from the laser catheter (for example, Patent document 1).

The laser catheter of Patent document 1 comprises an optical fiber which is built inside along the extending direction of a catheter tube, and an optical window which is provided in the outermost portion of a tip portion of the catheter tube, in an optically continuous manner with a tip of the optical fiber.

The optical window transmits through the irradiation light emitted from the tip of the optical fiber, and condenses a fluorescent light emitted from a photosensitive agent to the tip of the optical fiber.

For being thus configured, the laser catheter of Patent document 1 is capable of performing a photodynamic therapy with contacting the tip of the laser catheter with a target tissue for treatment.

### CITATION LIST

### PATENT DOCUMENT

Patent Document 1: JP 2012-147937 A

### DISCLOSURE OF THE INVENTION

### Problem to be Solved by the Invention

However, the laser catheter of Patent document 1 has a configuration in which a laser is emitted from a tip of the catheter tube, similarly as in a catheter for a radiofrequency ablation.

Accordingly, in a case of performing a treatment of arrhythmia by using a laser catheter of Patent document 1, an irradiation should be carried out to points one by one, and it is still necessary to connect the points in order to set a line for cutting off an abnormal electrical signal, similarly as in a radiofrequency catheter ablation.

The laser catheter of Patent document 1 was capable of carrying out a laser irradiation to a limited area, and in order to produce a line for interrupting abnormal electrical conduction, it was necessary to obtain a linear region for treatment by shifting an area to be irradiated, in order.

The present invention has been made in view of the above problems, and an object of the present invention is to provide a medical device with a light-emitting probe mounted to the outer side thereof, which is capable of producing lines for interrupting linear abnormal electrical conduction in a treatment of arrhythmia, and a kit for mounting a light-emitting probe for a medical device.

Another object of the present invention is to provide a kit for mounting a light-emitting probe, capable of easily externally mounting a light-emitting probe, by being combined with a medical device selected by an operator himself.

Still another object of the present invention is to provide a medical device with a light-emitting probe mounted to the outer side thereof, and a kit for mounting a light-emitting probe used in the medical device capable of easily converting an existing medical device into a medical device with a light-emitting probe mounted to the outer side thereof.

### Means for Solving Problem

According to the kit for mounting a light-emitting probe for a medical device of the present invention, the above problem is solved by being provided with a light transmission section that transmits a light from a light source, a light-emitting probe which is coupled to the light transmission section and emits a light transmitted by the light transmission section, and an externally mounting section for externally mounting the light-emitting probe to an outer portion of a medical device which is used by being inserted into a living body.

According to the medical device of the present invention, the above problem is solved by being provided with a light transmission section that transmits a light from a light source, a light-emitting probe which is coupled to the light transmission section and emits a light transmitted by the light transmission section, a medical device used by being inserted into a living body, and an externally mounting section for externally mounting the light-emitting probe on the outer portion of the medical device.

Being thus configured allows an operator to easily produce a medical device with a light-emitting probe by externally mounting a light-emitting probe to a medical device selected by the operator.

For example, catheters for radiofrequency ablation have been added with improvements for long time, and are satisfactory in operability or in close contact to a tissue, and in additional functions such as a 3D navigation, a contact sensor, etc. Therefore, by mounting a light-emitting probe on an outer side of every kind of catheters such as electrode catheters, ablation catheters, and the like, and sheaths, endoscopes, etc., it is possible to accomplish a catheter capable of emitting a light while exploiting functionalities (such as operability) of these medical devices.

In addition, since a probe to be mounted to an outer portion of a medical device is a light-emitting probe which uses a light, patterns of emission is not limited to points. By an emission pattern controlled to be in a linear shape, it is also possible to achieve the continuous region for treatment.

In addition, since the externally mounting section for externally mounting the light-emitting probe is provided to an outer portion of a medical device which is used by being inserted into a living body, an operator can easily mount a light-emitting probe on the medical device. By preparing light-emitting probes of plural types, it is possible to realize a very large number of types of medical devices with the light-emitting probes, in addition to alternatives of medical devices.

In addition, a mounting position and a mounting direction of the light-emitting probe may be arbitrarily set.

Here, a light emitting face of the light-emitting probe may be provided on a side face of the light-emitting probe, and may be continuously extended along an extending direction of the light-emitting probe.

For being thus configured, it becomes possible to control the pattern of emission to be in a linear shape, to accomplish a continuous region for treatment.

The pattern of emission may also be changed appropriately into a linear shape, a curvilinear shape, etc, by appropriately selecting a linear shaped or a curvilinear shaped catheter, etc. as the catheter to be mounted.

Here, the externally mounting section comprises a heat-shrinkable tube having a space capable of being inserted with the medical device inside prior to a heat shrinkage. The heat-shrinkable tube may be fixed to the light-emitting probe.

Being thus configured allows producing a medical device with a light-emitting probe only by inserting a medical device into the space of the heat-shrinkable tube and heating the tube, and thus, an operator can easily produce a medical device with a light-emitting probe in a simple operation.

Here, the externally mounting section may be capable of externally mounting a plural light-emitting probes to a single body of the medical device.

For being thus configured, it becomes possible to externally mounting the light-emitting probes on plural points on the external face of a medical device to allow a light emission from a light-emitting probe in an appropriate position, among the plural light-emitting probes. Accordingly, during a treatment, it is not necessary to consider a twist or a kink of a medical device, and thus, there is no need to finely adjust an operating handle at hand to move the light-emitting probe to an appropriate position. Here, the light emission may be controlled to be performed only from a light-emitting probe disposed on an appropriate position without being performed from the other light probes.

Here, the externally mounting section may comprise an opening that exposes an electrode formed on the external face of a medical device.

For being thus configured, even in a case of performing an electrophysiological examination or the like using a medical device provided with an electrode on an external face thereof, it is possible to measure a potential through the opening, and to exploit a function of the electrode.

Here, the externally mounting section may comprise a covering portion that covers a surface of the medical device, and the covering portion may comprise an anisotropic conductive body having a conductivity in the thickness direction, and an insulation in directions different from the thickness direction.

For being thus configured, even in a case of performing an electrophysiological examination or the like using a medical device provided with an electrode on an external face thereof, since the anisotropic conductive body of the covering portion which covers a surface of the medical device has a conductivity only in the thickness direction, it is possible to measure a potential on the basis of the conductivity, and to exploit a function of the electrode.

Here, the medical device may be a medical device selected from the group consisting of catheters, sheaths and endoscopes.

### ADVANTAGEOUS EFFECTS OF INVENTION

According to the present invention, an operator can easily produce a medical device with a light-emitting probe by externally mounting a light-emitting probe on a medical device selected by the operator.

For example, catheters for radiofrequency ablation have been added with improvements for long time, and are satisfactory in operability or in close contact to a tissue, and in additional functions such as a 3D navigation, a contact sensor, etc. Therefore, by mounting a light-emitting probe on an outer side of every kind of catheters such as electrode catheters, ablation catheters, and the like, and sheaths, endoscopes, etc., it is possible to accomplish a catheter capable of emitting a light while exploiting functionalities (such as operability) of these medical devices.

In addition, since a probe to be mounted to an outer portion of a medical device is a light-emitting probe which uses a light, patterns of emission is not limited to points. By an emission pattern controlled to be in a linear shape, it is also possible to achieve the continuous region for treatment.

In addition, since the externally mounting section for externally mounting the light-emitting probe is provided on an outer portion of a medical device which is used by being inserted into a living body, an operator can easily mount a light-emitting probe on the medical device. By preparing light-emitting probes of plural types, it is possible to realize a very large number of types of medical devices with the light-emitting probes, in addition to alternatives of medical devices.

Moreover, a mounting position and a mounting direction of the light-emitting probe may be arbitrarily set.

### BRIEF DESCRIPTION OF DRAWINGS

FIG. 1 is a partial external view of a light-emitting probe for a catheter according to Embodiment 1 of the present invention.
FIG. 2 is a partial external view of a laser catheter with the light-emitting probe according to Embodiment 1 of the present invention.
FIG. 3 is a partial cross-sectional view of the light-emitting probe for a catheter according to Embodiment 1 of the present invention.
FIG. 4 is an explanatory view showing a method of using the light-emitting probe for a catheter according to Embodiment 1 of the present invention.
FIG. 5 is a partial external view of the laser catheter with the light-emitting probe according to Embodiment 1 of the present invention.
FIG. 6 is a partial external view of an example of a variation of the light-emitting probe for a catheter according to Embodiment 1 of the present invention.
FIG. 7 is a partial external view of a light-emitting probe for a catheter according to Embodiment 2 of the present invention.
FIG. 8 is a partial external view of the laser catheter with the light-emitting probe according to Embodiment 2 of the present invention.
FIG. 9 is a partial external view of the light-emitting probe for a catheter according to Embodiment 2 of the present invention.
FIG. 10 is a partial external view of a light-emitting probe for a catheter according to Embodiment 3 of the present invention.
FIG. 11 is a partial external view of a laser catheter with the light-emitting probe according to Embodiment 3 of the present invention.
FIG. 12 is a partial external view of a light-emitting probe for a catheter according to Embodiment 4 of the present invention.
FIG. 13 is a partial external view of a laser catheter with the light-emitting probe according to Embodiment 4 of the present invention.
FIG. 14 is an explanatory view showing a connection state between an outer electrode and a catheter electrode in the laser catheter with the light-emitting probe according to Embodiment 4 of the present invention.
FIG. 15 is a partial external view of a light-emitting probe for a catheter according to Embodiment 5 of the present invention.
FIG. 16 is a partial external view of a laser catheter with the light-emitting probe according to Embodiment 5 of the present invention.
FIG. 17 is an explanatory view showing a status of use of the laser catheter with the light-emitting probe according to Embodiment 5 of the present invention.
FIG. 18 is a partial external view of a laser catheter with the light-emitting probe according to another embodiment of the present invention.
FIG. 19 is a cross-sectional explanatory view showing a structure of an anisotropic conductive body used in a cover in the variation of the laser catheter with the light-emitting probe according to Embodiment 4 of the present invention.
FIG. 20 is a partial external view of the light-emitting probe for an endoscope and an endoscope according to another embodiment of the present invention.

### DETAILED DESCRIPTION OF THE INVENTION

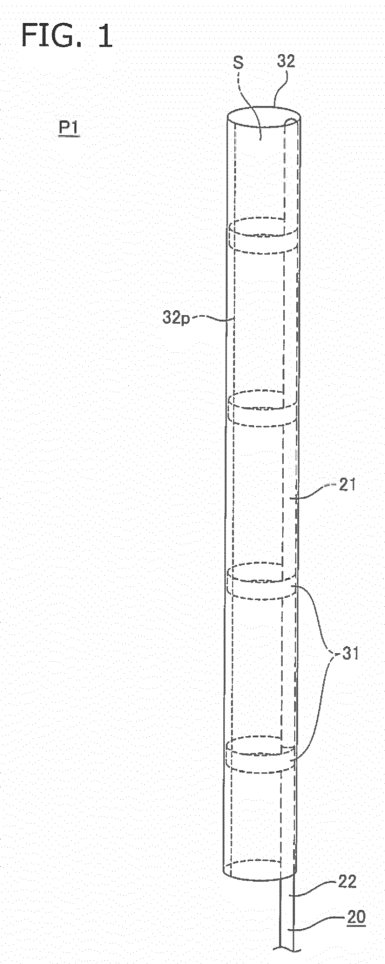

Hereinbelow described with reference to Figs. 1 to 20 are light-emitting probes P1 to P5 for a laser catheter according to Embodiments 1, 3, 5, 7 and 9 of the present invention; laser catheters 1 to 5 as medical devices according to Embodiments 2, 4, 6, 8 and 10 which is provided with these light-emitting probes for laser catheter; and the emitting probe P6 for an endoscope according to Embodiment 11.

In this connection, although the embodiments describe a laser catheter and an endoscope as the medical device, the medical device is not limited thereto, but only has to be those introduced into a living body to a target tissue to be treated, such as a sheath or other medical tubes.

Similarly, although the each embodiment in the present specification describes an example which uses the medical device with the light-emitting probe mounted thereon in a treatment of arrhythmia in which a line for interrupting abnormal electrical conduction is produced by a photodynamic therapy; and an example which uses the medical device with the light-emitting probe mounted thereon in a photodynamic therapy under endoscope, for cancers in a pancreas or a biliary tract, using a very thin small-diameter endoscope, such as a biliary tract endoscope (outer diameter of 1 to 3 mm) or a pancreas endoscope (outer diameter of 1 to 2. 5 mm), the present invention is not limited thereto.

The medical device of the present invention may be used in a morbid state where it is possible to use a medical device provided with a light-emitting probe, typified by a laser catheter. For example, the medical device of the present invention may be used in a photodynamic therapy for cancers, infectious diseases or arteriosclerosis, or in a treatment of thrombosis and the like in which a laser catheter is used. The medical device of the present invention may also be used in any case where a laser irradiation or a laser measurement is performed.

In addition, in the present embodiments, a heat-shrinkable tube or the like is used as the mounting section of the embodiments. However, the mounting section is not limited thereto, but a clip, a ring or like may also be used.

### << Embodiment 1: Light-emitting probe P1 for a catheter >>

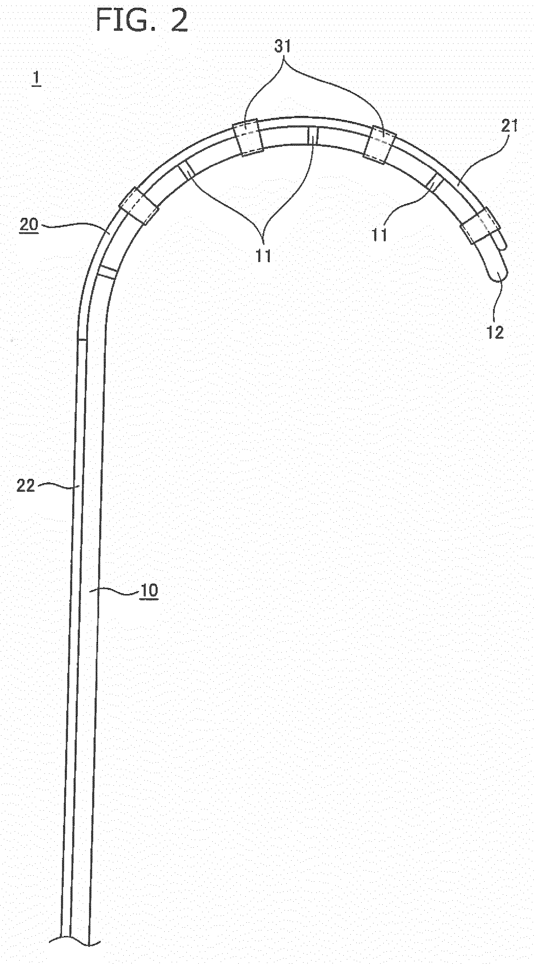

The light-emitting probe P1 for a catheter of the present embodiment comprises a light-emitting probe member 20, and a ring tube 31 and a stripping tube 32 having the light-emitting probe member 20 inserted therein as shown in Fig. 1, and provided as a kit for producing the laser catheter 1 with a light-emitting probe of Fig. 2.

In other words, it is possible to produce the laser catheter 1 with a light-emitting probe of Fig. 2, by inserting a catheter 10 into the ring tube 31 and the stripping tube 32 of the light-emitting probe P1 for a catheter, and shrinking the ring tube 31 and the stripping tube 32 by heating, and separating the stripping tube 32.

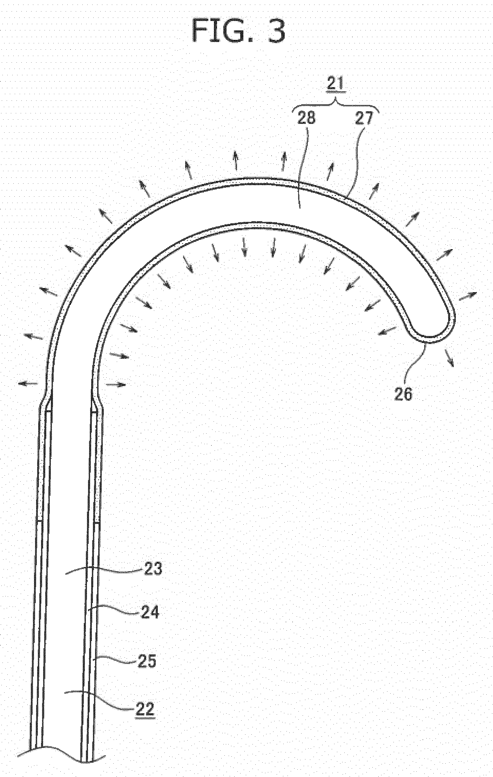

The light-emitting probe member 20 preferably has a diameter greater than a minimum diameter of a typical optical fiber and smaller than a diameter of about a catheter diameter, and preferably comprises a substantially cylindrical body with a diameter of 0.125 to 2.5 mm, preferably 0.25 to 1 mm. The light-emitting probe member 20 comprises an optical fiber cable 22 and a laser probe 21 which is continuously provided to a tip of the optical fiber cable 22, as shown in Fig. 3.

The optical fiber cable 22 is configured by covering a long core 23 with a cladding 24 and a covering 25 in order, as shown in Fig.3. The optical fiber cable 22 comprises the core 23 which comprises polymethyl methacrylate, polystyrene, polycarbonate or the like, and the cladding 24 which comprises plastic optical fibers comprising a fluorinated polymer or the like. Although it is preferred to use a plastic optical fiber which is strong in bending, a glass optical fiber may also be used.

Since the light-emitting probe member 20 is fixed to the catheter 10, the light-emitting probe member 20 is to use an operability of the catheter 10, without need of operability itself. However, the light-emitting probe member 20 may be imparted with an operability, and the light-emitting probe member 20 may also be formed with a shape memory material.

The laser probe 21comprises a light diffusing body 28 which is formed such that the cladding 24 and the covering 25 are removed from the optical fiber cable 22 to expose the core 23 over a predetermined length from a tip portion 26; and a resin layer 27 which covers the outer periphery of the light diffusing body 28, as shown in Fig 3.

The light diffusing body 28 formed in an integral manner with the core 23 is formed by performing a sandblast processing on the core 23, to uniformize emission lights to the side directions with angles with respect to the longitudinal direction of the light diffusing body 28. In this connection, without limitation, the emission lights to the side directions may also be uniformized by providing the light diffusing body 28 with a hollow portion in the center, and a reflecting mirror on an inner face of the hollow portion, or by providing indentations on the inner face.

The resin layer 27 is formed, for example, by being applied to an acrylic ultraviolet curing resin in which a fine powder of quartz are dispersed, and cured with ultraviolet light.

To an end portion in the side opposite to the laser probe 21 of the optical fiber cable 22, a connector (not shown) is fixed, by which the laser probe 21 is configured to be connectable to a laser generator (not shown) which stores a laser generating source (not shown).

Incidentally, the light diffusing body 28 is not limited to the type of Fig. 3, but other types may also be used.

Methods for composing the light diffusing body 28 are roughly classified into a case of extending the core 23 of the optical fiber cable 22 to compose the light diffusing body 28, and a case of providing a light diffusing body 28 as a separate body from the core 23, and both of the light diffusing bodies 28 can be used as the light diffusing body 28 of the present embodiment.

The former includes a case that the core 23 constitutes the diffusing substance itself and a case that the core 23 does not constitute the diffusing substance itself. Concretely, it is roughly classified into a transmission light leakage method (a method of partially exposing the core 23 by making faint scratches on the cladding 24, or a method of constituting a leakage by bending, etc.) and a method of using a diffusive substance.

The transmission light leakage method includes scratch processing (sandblasting, stamping, solvent treatment, etc.), fiber bragg grating (FBG), micro-bending, and the like.

Further, methods of using the diffusive substance include a method in which the diffusive substance is put within the core 23/the cladding 24, and a method in which the core 23 is exposed and the diffusive substance is put within the covering 25, and so on. In this connection, sandblasting is a method of spraying fine particles, and therefore, falls under the method of using the diffusion substance.

The latter, which is the case of providing a light diffusing body 28 as a separate body from the core 23, includes a case of using an optical element which is different from the core 23 as the light diffusing body 28. Examples therefor include using an optical element such as a polyhedral prism, SELFOC (registered trademark) lens (gradient index lens) or the like, as the light diffusing body 28.

The ring tube 31 is formed with a heat-shrinkable tube for medical use of a transparent fluoropolymer or the like, cut into a ring shape, as in Fig. 1. The ring tube 31 is constructed with a substance which is soft to a degree that the original functions of the catheter 10 and the light-emitting probe member 20 are not lost.

The stripping tube 32 is a heat-shrinkable tube for medical use of a transparent fluoropolymer or the like, and as shown in Fig. 1, provided with linear thin portions 32p over the entire length in the longitudinal direction thereof. These thin portions 32p are configured to allow the stripping tube 32 to be torn in the longitudinal direction, for being thinner and more vulnerable than the other portions of the stripping tube 32.

Thickness of the ring tube 31 is about 2/3 mm or less, and preferably is about 1/3 mm or less.

The stripping tube 32 is configured to have an inner diameter before a heat shrinkage a little larger than an outer diameter of the ring tube 31 before a heat shrinkage.

The outer face of the ring tube 31 is partially fixed to the inner face of the stripping tube 32, weakly with an adhesive. Strength of this fixation is in a degree that allows the stripping tube 32 and the ring tube 31 to be separated by hand, when the stripping tube 32 is torn along the thin portions 32p.

In this embodiment, the stripping tube 32 was constructed with a continuous seamless tubular body. However, without limitation, the stripping tube 32 may also be configured in such a manner that a sheet of strip-shaped long body which comprises a heat-shrinkable resin is configured into a tubular body having a shape of sushi roll, such that the both ends in the width direction are bonded with each other in an overlapped manner.

To a portion of the inner face of the ring tube 31, the outer face of the light-emitting probe member 20 is fixed with an adhesive.

The light-emitting probe P1 for a catheter comprises a tubular space S in a region adjacent to the light-emitting probe member 20 in the portion surrounded by the ring tube 31 and the stripping tube 32, as shown in Fig. 1.

This space S is used as a space, into which an operator who is a user of the light-emitting probe P1 for a catheter inserts a catheter 10 selected by himself.

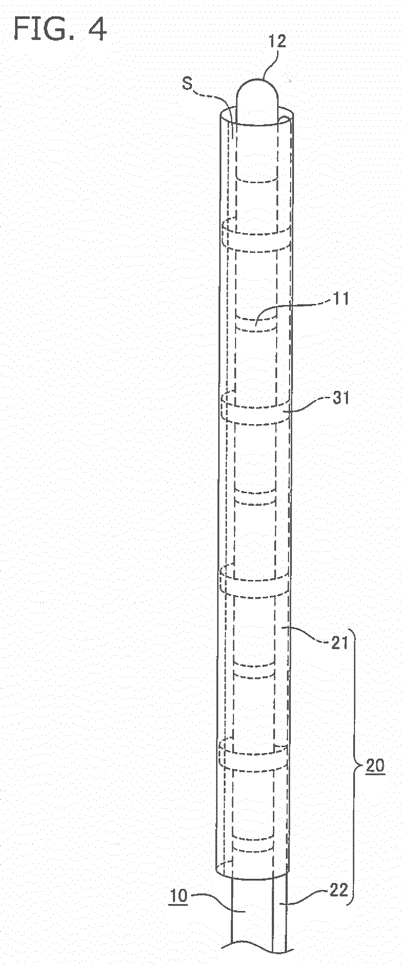

The stripping tube 32 is colored to a degree that it is possible to visually confirm the catheter 10 inserted into the space S from outside, for example, colorless and transparent or colored and transparent. For being thus configured, when the catheter 10 is inserted into the space S, it becomes easily possible to visually confirm a foreign substance such as dust if put between the catheter 10 and the stripping tube 32. In addition, if the stripping tube 32 and the ring tube 31 are made transparent, semitransparent, or nontransparent with colored with a color different from that of the stripping tube 32, it is possible to use it a mark for positioning the ring tube 31 so as not to overlap with a ring-shaped electrode 11 and a tip electrode 12 of the catheter 10, when the catheter 10 is inserted into the space S, as in Fig. 4.

The ring tube 31 and the stripping tube 32 may be formed with an antibacterial material.

The light-emitting probe P1 for a catheter of the present embodiment is produced by the following process.

In the first place, a colored heat-shrinkable tube is formed with a heat-shrinkable material by a publicly known method, and the obtained tube is cut through lines perpendicular to the longitudinal direction of the tube to produce plural ring tubes 31.

Then, on an outlet side of an extruder for the stripping tube 32 provided with a mandrel and an extrusion head, a long shaft having a diameter a litter smaller than that of the mandrel is disposed.

Through this long shaft, the plural ring tubes 31 are inserted with being spaced from one another at predetermined intervals.

In this state, the stripping tube 32 comprising a transparent heat-shrinkable tube is formed by using a publicly known extruder, in such a manner that the stripping tube 32 is extruded so as to have the inside thereof inserted through by the long shaft and the plural ring tubes 31.

Subsequently, the positions where the ring tubes 31 and the stripping tube 32 are overlapped with each other are irradiated with a laser from outside the stripping tube 32 to an extent that the ring tubes 31 and the stripping tube 32 do not shrink, to thereby weld and fix the ring tubes 31 and the stripping tube 32.

Then, in the light-emitting probe member 20, a position corresponding to a position of a ring tube 31 in the tip side is applied with a transparent adhesive; the light-emitting probe member 20 inserts through the ring tubes 31 and the stripping tube 32; and the light-emitting probe member 20 is fixed to the ring tube 31.

As the above, the light-emitting probe P1 for a catheter is completed.

### <<Use of Light-emitting probe P1 for a catheter >>

In the next place, a method of using the light-emitting probe P1 for a catheter of Fig. 1 by an operator who is a user will be described.

Light-emitting probes P1 for a catheter is provided in the state shown in Fig. 1.

An operator who is a user of the light-emitting probe P1 for a catheter selects a catheter 10 to be used in combination with the light-emitting probe member 20, according to a method of treatment or a target site. The selected catheter 10 is inserted into the space S of Fig. 1, such that the tip thereof positions in the tip side of the light-emitting probe member 20, and arranged as in Fig. 4.

In a case where the catheter 10 is an electrode catheter, the catheter is disposed such that the ring-shaped electrode 11 and the tip electrode 12 do not face the ring tube 31.

Subsequently, the ring tube 31 and the stripping tube 32 are shrunk by heating, so that the catheter 10 and the light-emitting probe member 20 are fixed to each other in a direction perpendicular to the longitudinal direction.

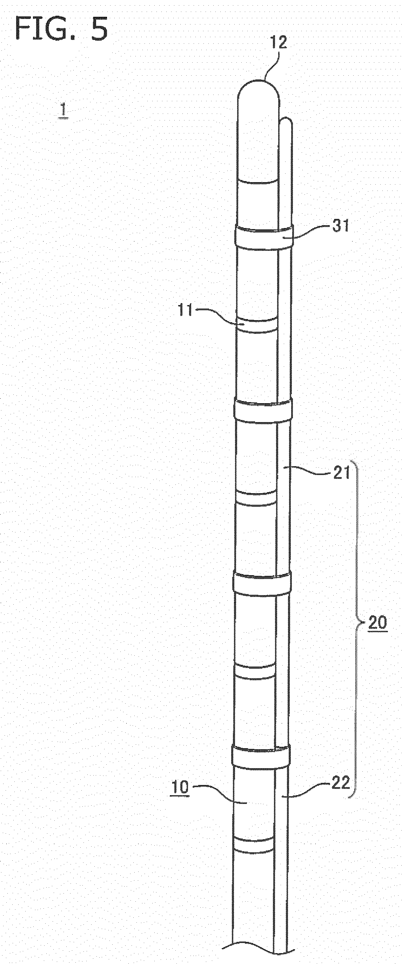

Then, the stripping tube 32 is torn along the thin portions 32p and removed with caring not to remove the ring tube 31 with the stripping tube 32, and thus the laser catheter 1 with the light-emitting probe of Fig. 5 is completed.

In the laser catheter 1 with a light-emitting probe of Fig. 5, the catheter 10 and the light-emitting probe member 20 are fixed to each other by the ring tubes 31.

Generally, when an electrode of a catheter has about 300° of the total circumference thereof exposed, there is no hindrance in measuring an electric potential. Therefore, it is preferred that the laser catheter 1 with a light-emitting probe of Fig. 5 is arranged such that about 300° or more in the circumferential direction of the ring-shaped electrodes 11 is exposed from the light-emitting probe member 20.

Incidentally, although the present embodiment described an example in which the light-emitting probe P1 for a catheter was provided with a single light-emitting probe member 20, the light-emitting probe P1 for a catheter may be provided with plural light-emitting probe members 20, such as two, three, and so on.

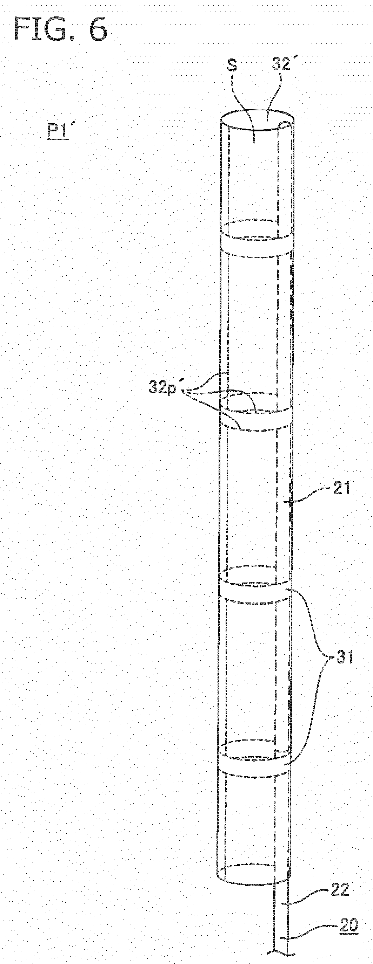

In the present embodiment, the externally mounting section to externally mounting a light-emitting probe member 20 on the outer portion of the catheter 10 is configured into a two-layered structure in which the stripping tube 32 is bonded to an outer side of the ring tube 31. However, the externally mounting section may also be configured with a heat-shrinkable tube 32' in a single-layered structure, in which the externally mounting section can be stripped along thin portions 32p' with leaving the ring tubes 31', as in the light-emitting probe P1' for a catheter shown in Fig.6.

The thin portions 32p' are portions configured to be thinner and more vulnerable than the other portions of the heat-shrinkable tube 32', which comprise a pair of circulars which form a ring-shaped ring tube 31' perpendicular to the longitudinal direction of the heat-shrinkable tube 32', and a straight line which connects the ring tubes 31' adjacent to one another in the longitudinal direction, which are formed alternately over the entire length in the longitudinal direction of the heat-shrinkable tube 32'.

The light-emitting probe P1' for a catheter is provided in the state shown in Fig. 6. An operator who is a user inserts the catheter 10 into the space S, such that the tip thereof positions in the tip side of the light-emitting probe member 20. Then, after the heat-shrinkable tube 32' is shrunk by heating, the heat-shrinkable tube 32' is torn along the thin portions 32p' to remove portions other than the ring tubes 31', to thus complete the laser catheter 1 with a light-emitting probe shown in Fig. 5.

As for other configurations of the light-emitting probe P1' for a catheter, explanation is omitted, because they are similar to those of the light-emitting probe P1 for a catheter.

In this connection, it is preferred that a pattern of the thin portions 32p' is formed such that the distances between the ring tubes 31' adjacent to one another in the longitudinal direction are substantially the same with the spaces of the ring-shaped electrodes 11 of a catheter 10 expected to be used. However, spaces of the ring-shaped electrodes 11 may be identical spaces or may be made at random.

In the present embodiment, a pattern of the ring tubes 31' to be left after the stripping along the thin portions 32p' was formed in a direction perpendicular to the longitudinal direction of the catheter 10. However, the pattern may be formed oblique to the longitudinal direction of the catheter 10. For example, if the pattern is formed into an X-shaped lattice state, such that the portions to be removed form rhombuses relative to the longitudinal direction of the catheter 10, the remaining portions after the rhombus portions are removed form an oblique lattice state, for which, there is no need of considering positions of electrodes.

### <<Embodiment 2: Laser catheter 1 with a light-emitting probe 1>>

The Embodiment 1 described an example in which the laser catheter 1 with a light-emitting probe of Fig. 2 or Fig. 5 was produced by an operator who is a user by combining a light-emitting probe P1 for a catheter with a catheter 10 selected by himself. However, the present invention may be provided as in the embodiment of the laser catheter 1 with a light-emitting probe of Fig. 2 or Fig. 5 in a manner not limited thereto.

The laser catheter 1 with a light-emitting probe comprises a publicly known catheter 10 comprising a hollow soft tubular body, and the light-emitting probe member 20, fixed with each other by the ring tube 31, as shown in Fig. 2 or Fig. 5.

The catheter 10 of the present embodiment comprises a publicly known electrode catheter which is configured to have a bendable tip side which can be curved into a shape of letter J, as shown in Fig. 2. The tip side thereof is provided with a tip electrode 12 which is fixed to the tip, and ring-shaped electrodes 11 in plural numbers fixed to the outer periphery face in the tip side at a predetermined intervals.

Inside the catheter 10, a tractive wire and a deflection structure (not shown) for curvedly bending the tip side, and lead wires connected to each of the tip electrode 12 and the ring-shaped electrodes 11 are arranged. To the end portion in the side opposite to the tip side of the catheter 10, a control handle (not shown) for controlling the tractive wire and the deflection structure (not shown) to control the catheter 10 is connected.

As for other configurations of the present embodiment, explanation is omitted, because they are similar to each configuration of the light-emitting probe P1 for a catheter of Embodiment 1.

### << Embodiment 3: Light-emitting probe P2 for a catheter >>

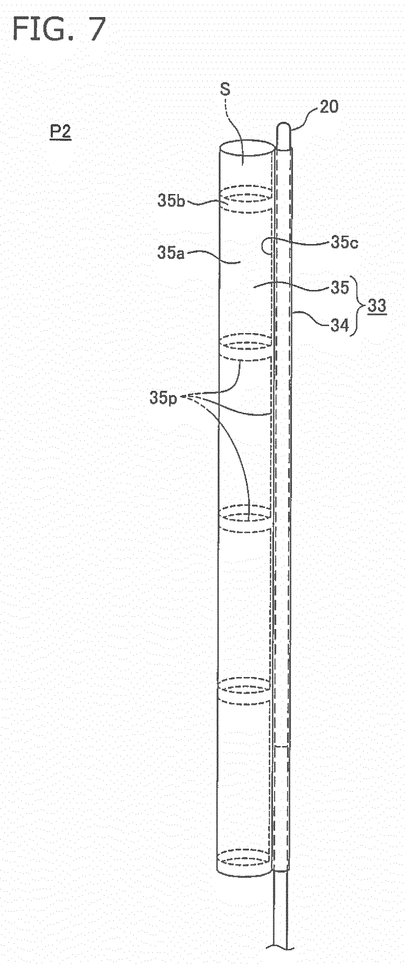

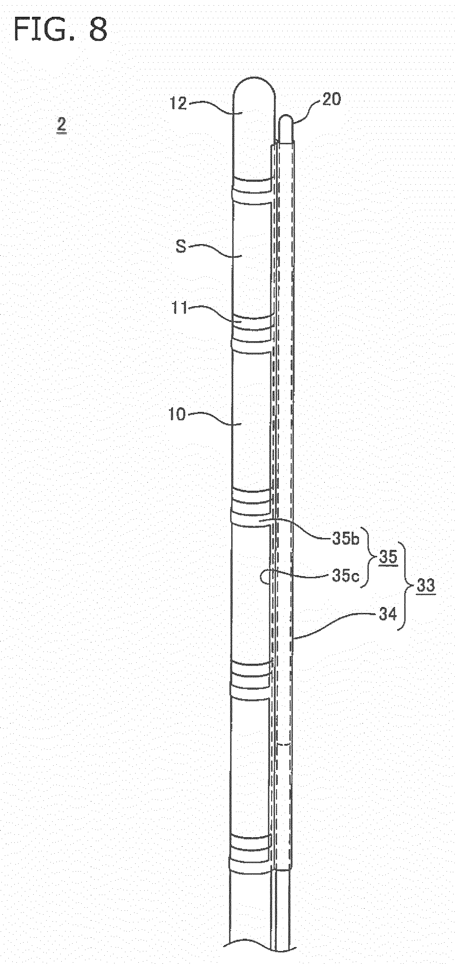

Another example of the light-emitting probe for a catheter in the present invention will be described on the basis of Fig. 7 and Fig. 8.

The light-emitting probe P2 for a catheter of the present embodiment is provided with a light-emitting probe member 20 and a double tube 33 with a cross section in a shape of letter 8, having the light-emitting probe member 20 inserted therein, as shown in Fig. 7, and provided as a kit for producing the laser catheter 2 with a light-emitting probe of Fig. 8.

The double tube 33 comprises a first rube 34, into which the light-emitting probe member 20 is inserted and fixed, and a second tube 35 which forms a space S for the catheter 10 to be inserted through, which are weld to each other by the outer surfaces thereof along a connecting portion 35c of the second tube 35.

The first tube 34 is formed with a heat-shrinkable tube for medical use of a transparent fluoropolymer or the like, which has been shrunk by heating with the light-emitting probe member 20 inserted inside, and is in a state that the light-emitting probe member 20 is fixed inside.

The second tube 35 is formed with a heat-shrinkable tube for medical use of a transparent fluoropolymer or the like, which has not been heated, and is in a state not shrunk.

The first tube 34 and the second tube 35 are constructed with a material which is soft to an extent that the original functions of the catheter 10 and the light-emitting probe member 20 are not lost.

In the second tube 35, linear thin portions 35p which are configured to be thinner and more vulnerable than the other portions are formed, as shown by the broken line in Fig. 7. The thin portions 35p are formed so as to partition the side surface of the second tube 35 into regions to be stripped off 35a surrounded by plural rectangular patterns arranged in a row at regular intervals in the longitudinal direction of the second tube 35, and the other regions. The regions other than the regions to be stripped off 35a comprise plural ring portions 35b formed into a ring shape perpendicular to the longitudinal direction of the second tube 35, with being spaced from one another at predetermined intervals in the longitudinal direction, and a connecting portion 35c which extends in a straight line along the longitudinal direction of the second tube 35 and connects the plural ring portions 35b in the longitudinal direction of the second tube 35, as shown in Fig. 7. The connecting portion 35c serves as a margin on which the first tube 34 is connected to the second tube 35.

Since the second tube 35 thus comprises the thin portions 35p which draw the rectangle patterns, when the catheter 10 is inserted into the second tube 35, then the tube is shrunk by heating, and thereafter, the second tube 35 is torn along all the thin portions 35p, it is possible that the regions to be stripped off 35a surrounded by the rectangle patterns in the second tube 35 are stripped off with leaving only the plural ring portions 35b and the connecting portion 35c in a state connected to the first tube 34.

Incidentally, the pattern drawn by the thin portions 35p is not limited to rectangles, but may be any shape, provided that the shape allows at least a portion of the ring-shaped electrodes 11 and of the tip electrode 12 of the catheter 10 to be exposed.

The light-emitting probe P2 for a catheter of the present embodiment is produced by the following process.

In the first place, a first tube 34 and a second tube 35 are formed with a heat-shrinkable material, by a publicly known method. Then, thin portions 35p are formed on the second tube 35 by a publicly known method. Incidentally, the thin portions 35p may be simultaneously formed at the time the second tube 35 is formed.

Then, after a light-emitting probe member 20 is inserted into the first tube 34, only the first tube 34 is shrunk by heating to fix the light-emitting probe member 20 inside the first tube 34.

Thereafter, a side face of the first tube 34 is contacted with the connecting portion 35c of the second tube 35, and the contacted portion is laser-welded, to thus form the double tube 33 in which the first tube 34 and the second tube 35 are bonded with each other on side faces thereof such that the cross section is formed into a shape of letter 8.

As the above, the light-emitting probe P2 for a catheter is completed.

Incidentally, in the present embodiment, the second tube 35 is configured into a single layered structure capable of being stripped off along the thin portions 35p with leaving the ring portions 35b and the connecting portion 35c. However, the second tube 35 may also be configured into a two-layered structure similarly to Fig. 1. In this case, the two-layered structure is preferably configured by bonding a tubular stripping tube (not shown) on an outer side of a ring-shaped ring tube (not shown) having a shape identical to that of the ring portion 35b, at a bonding strength of an extent which allows the tubular stripping tube to be separated by hand. Here, it is preferred that the stripping tube (not shown) comprises a pair of linear thin portions extending in the longitudinal direction in the both sides of the connecting portion 35c, along which the stripping tube (not shown) is torn so that a portion having a shape identical to the shape of the connecting portion 35c is left and the other portions are stripped off.

### <<Use of light-emitting probe P2 for a catheter >>

In the next place, a method of using the light-emitting probe P2 for a catheter of Fig. 7 by an operator who is a user will be described.

The light-emitting probe P2 for a catheter is provided in the state shown in Fig. 7.

An operator who is a user of the light-emitting probe P2 for a catheter disposes a selected catheter 10 in the space S of Fig. 7, as in Fig. 8.

Subsequently, a second tube 35 is shrunk by heating, so that the catheter 10 is fixed inside the second tube 35, and then the second tube 35 is torn along the thin portions 35p to remove the regions to be stripped off 35a with leaving the ring portions 35b and the connecting portion 35c, to thus complete the laser catheter 2 with a light-emitting probe of Fig. 8.

### <<Embodiment 4: Laser catheter 2 with a light-emitting probe>>

The embodiment 3 described an example in which the laser catheter 2 with a light-emitting probe of Fig. 8 was produced by an operator who is a user by combining a light-emitting probe P2 for a catheter with a catheter 10 selected by himself. However, the present invention may be provided as in the embodiment of the laser catheter 2 with a light-emitting probe of Fig. 8 in a manner not limited thereto.

The laser catheter 2 with a light-emitting probe comprises a publicly known catheter 10 and the light-emitting probe member 20, fixed with each other by the double tube 33, as shown in Fig. 8.

As for other configurations of the present embodiment, explanation is omitted, because they are similar to each configuration of the light-emitting probes P1 and P2 for a catheter of Embodiments 1 and 3.

The Embodiments 3 and 4 described examples in which the light-emitting probe P2 for a catheter is provided with a single light-emitting probe member 20. However, the light-emitting probe P2 for a catheter may comprise plural light-emitting probe members 20, such as two, three, and so on.

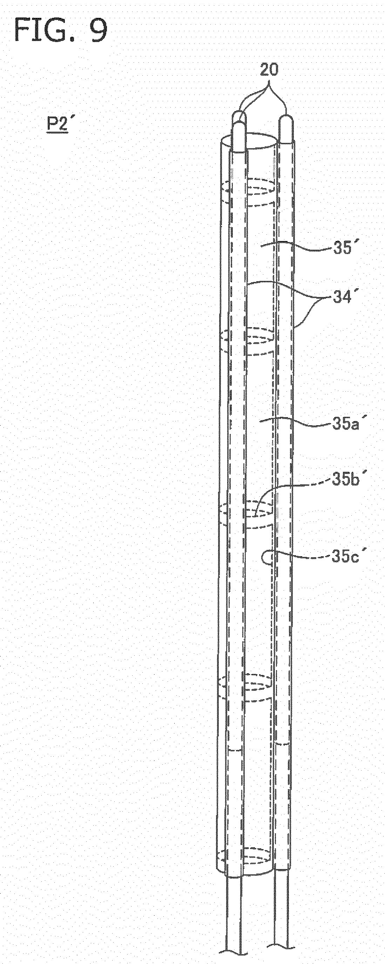

Fig. 9 shows a light-emitting probe P2' for a catheter which is provided with three light-emitting probe members 20.

In the light-emitting probe P2' for a catheter, three first tubes 34' are fixed to the side face of the second tube 35 at regular intervals in the circumferential direction, as shown in Fig. 9.

To each of the first tubes 34', a light-emitting probe member 20 is inserted and fixed.

In the second tube 35', linear thin portions 35p are formed, as shown by the broken line in Fig. 9.

The thin portions 35p' are formed so as to draw plural rectangular patterns arranged in three rows at predetermined intervals in the longitudinal direction of the second tube 35' on the side face of the second tube 35' to partition the side face into regions to be stripped off 35a' surrounded by the plural rectangular patterns, and the other regions.

Regions other than the regions to be stripped off 35a' comprises plural ring portions 35b' formed into a ring shape perpendicular to the longitudinal direction of the second tube 35', with being spaced from one another at predetermined intervals in the longitudinal direction, and a connecting portion 35c' which extends in a straight line along the longitudinal direction of the second tube 35' to connect the plural ring portions 35b' in the longitudinal direction of the second tube 35', as shown in Fig 9. In the example of Fig. 9, three connecting portions 35c' are provided at regular intervals in the circumferential direction of the second tube 35' to partition the regions to be stripped off 35a' into three portions in the circumferential direction. Accordingly, the ring portions 35b' and the connecting portion 35c' form a lattice pattern on the side face of the second tube 35'.

The three connecting portions 35c' each have a first tubes 34' welded and fixed thereto.

In this connection, the number of the connecting portion 35c' and the second tube 35' is not limited to three, but may be two, four or more. However, in order to maintain the functions of the ring-shaped electrodes 11 and the tip electrode 12 of the catheter 10, it is preferred that the number is about 2 to 3.

By thus providing the connecting portions 35c' and the second tubes 35' in plural numbers, it becomes possible to externally mount plural light-emitting probe members 20 on a catheter 10, and it becomes possible that desired portions are easily irradiated with light, regardless of presence or absence of twist of the catheter 10 or an angle of the catheter 10 within a living body. Furthermore, it becomes also possible that the light emission may be controlled to be performed only from a light-emitting probe member 20 disposed at an appropriate position without being performed from the other light probe members 20.

In this connection, in the present embodiment, the second tube 35' is configured into a single layered structure capable of being stripped off along the thin portions 35p', with leaving the ring portions 35b and the connecting portion 35c'. However, the second tube 35' may also be configured into a two-layered structure similarly to Fig. 1. In this case, the two-layered structure is preferably configured by bonding a tubular stripping tube (not shown) on an outer side of a ring-shaped ring tube (not shown) having a shape identical to that of the ring portion 35b, at a bonding strength of an extent which allows the tubular stripping tube to be separated by hand. Here, it is preferred that the stripping tube (not shown) comprises a pair of linear thin portions extending in the longitudinal direction in the both sides of the connecting portion 35c', along which the stripping tube (not shown) is torn so that a portion having a shape identical to the shape of the connecting portion 35c' is left and the other portions are stripped off.

### << Embodiment 5: Light-emitting probe P3 for a catheter >>

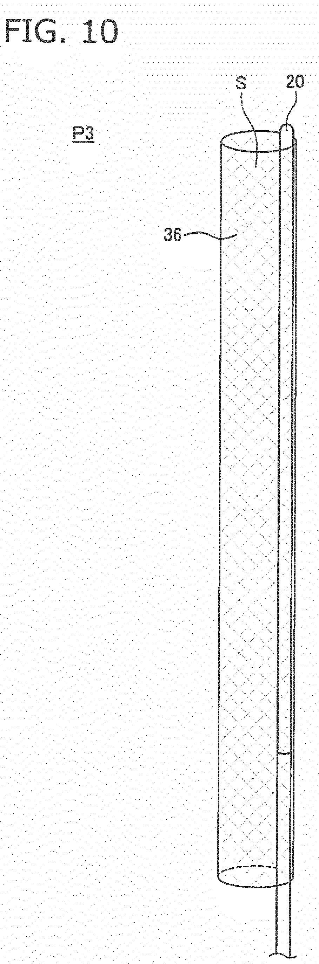

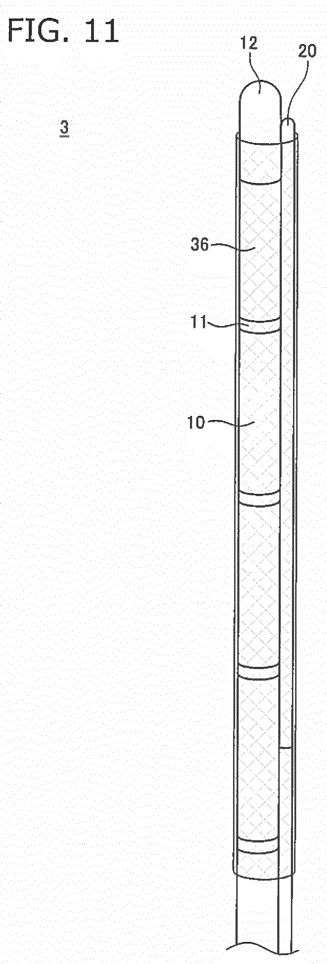

Still another example of the light-emitting probe for a catheter in the present invention will be described on the basis of Fig. 10 and Fig. 11.

The light-emitting probe P3 for a catheter of the present embodiment is provided with a light-emitting probe member 20 and a mesh tube 36 having the light-emitting probe member 20 inserted therein as shown in Fig. 10, and provided as a kit for producing the laser catheter 3 with a light-emitting probe of Fig. 11.

The mesh tube 36 is formed with a transparent fluoropolymer or the like and comprises a heat-shrinkable-type mesh tube which is thermally shrunk by heating. The mesh tube 36 is constructed with a material which is soft to an extent that the original functions of the catheter 10 and the light-emitting probe member 20 are not lost. Thickness of the mesh tube 36 is about 2/3 mm or less, and preferably is about 1/3 mm or less.

Roughness of mesh of the mesh tube 36 is in a degree that would not cause a hindrance in measuring an electric potential of the tip electrode 12 and the ring-shaped electrodes 11 of the catheter 10 after a heat shrinkage. For example, a mesh with an aperture ratio of about 30 to 90% is used.

To a portion of the inner face of the mesh tube 36, an outer face of the light-emitting probe member 20 is fixed with an adhesive.

The light-emitting probe P3 for a catheter is provided with a tubular space S in a region adjacent to the light-emitting probe member 20 in the portion surrounded by the mesh tube 36, as shown in Fig. 10, and this space S is used as a space into which an operator who is a user of the light-emitting probe P3 for a catheter inserts a catheter 10 selected by himself.

The light-emitting probe P3 for a catheter of the present embodiment is produced by the following process.

In the first place, a mesh tube 36 is formed with a heat-shrinkable material by a publicly known method.

After inserting a light-emitting probe member 20 into the mesh tube 36, the mesh tube 36 and the light-emitting probe member 20 are laser-welded to complete the light-emitting probe P3 for a catheter.

### <<Use of light-emitting probe P3 for a catheter >>

In the next place, a method of using the light-emitting probe P3 for a catheter of Fig. 10 by an operator who is a user will be described.

Light-emitting probe P3 for a catheter is provided in the state shown in Fig. 10.

An operator who is a user of the light-emitting probe P3 for a catheter disposes a selected catheter 10 in the space S of Fig. 10.

Subsequently, the mesh tube 36 is shrunk by heating and the catheter 10 is fixed in the mesh tube 36, to thus complete the laser catheter 3 with a light-emitting probe of Fig. 11.

### «Embodiment 6: Laser catheter 3 with a light-emitting probe»

The embodiment 5 described an example in which the laser catheter 3 with a light-emitting probe of Fig. 11 was produced by an operator who is a user by combining a light-emitting probe P3 for a catheter with a catheter 10 selected by himself. However, the present invention may be provided as in the embodiment of the laser catheter 3 with a light-emitting probe of Fig. 11 in a manner not limited thereto.

The laser catheter 3 with a light-emitting probe comprises a publicly known catheter 10 and the light-emitting probe member 20, fixed with each other by the mesh tube 36, as shown in Fig. 11.

As for other configurations of the present embodiment, explanation is omitted, because they are similar to each configuration of the light-emitting probes P1 and P3 for a catheter of Embodiments 1 and 5.

### << Embodiment 7: Light-emitting probe P4 for a catheter >>

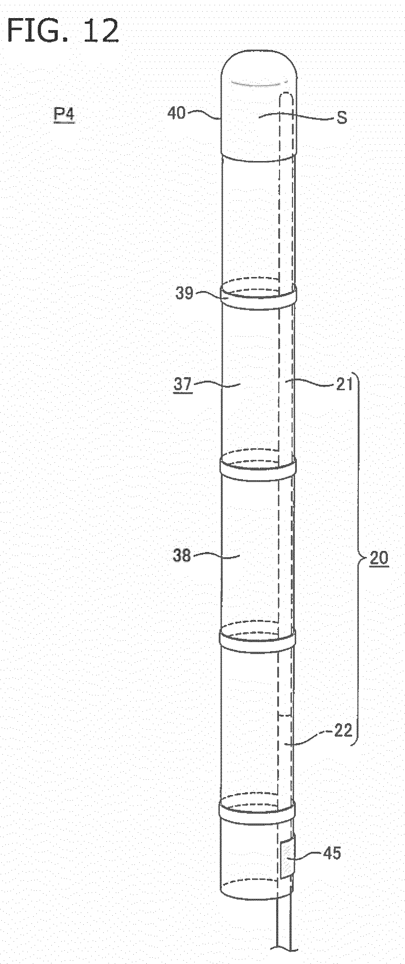

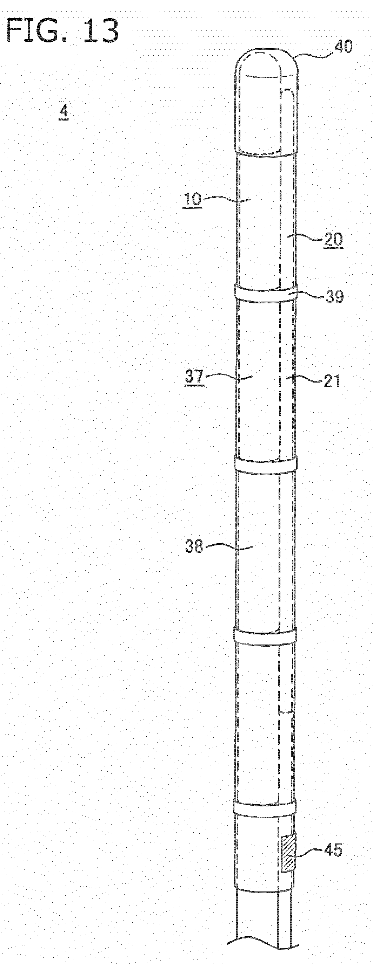

Still another example of the light-emitting probe for a catheter in the present invention will be described on the basis of Fig. 12 and Fig. 13.

The light-emitting probe P4 for a catheter of the present embodiment is provided with a light-emitting probe member 20 and a cover 37 having the light-emitting probe member 20 inserted therein as shown in Fig. 12, and provided as a kit for producing the laser catheter 4 with a light-emitting probe of Fig. 13.

The cover 37 is provided with a transparent tube 38 having a bag-shape formed by closing an end of a cylindrical body, outer side ring-shaped electrodes 39 which are mounted to predetermined positions in the longitudinal direction of the outer side of the transparent tube 38 at predetermined intervals; an outer side tip electrode 40 mounted to an end in the outer side of the transparent tube 38, and a marker 45 provided at a position in the circumferential direction of the outer periphery of the cover 37.

The transparent tube 38 is constructed with a publicly known medical use tube of silicone or the like, and has a thickness which allows accommodating the light-emitting probe member 20 and the catheter 10 kept in contact with the inner periphery of the catheter 10. The transparent tube 38 is constructed with a material which is soft to an extent that the original functions of the catheter 10 and the light-emitting probe member 20 are not lost. Thickness of the transparent tube 38 is about 2/3 mm or less, and preferably is about 1/3 mm or less.

The outer side ring-shaped electrodes 39 are formed into a ring-shape with a diameter a little larger than the diameter of the transparent tube 38, and comprises ring-shaped electrodes formed with a publicly-known material such as platinum.

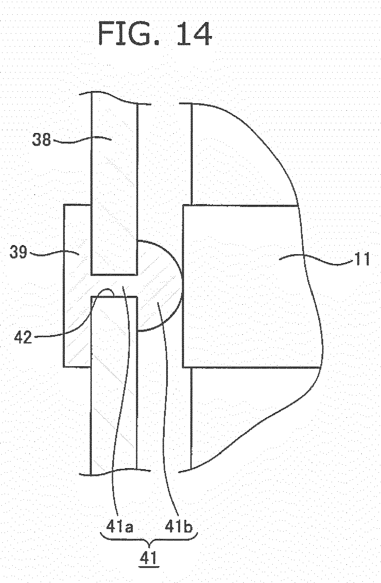

On the inner face of the outer ring-shaped electrodes 39, projections 41 which project inward are formed on plural points, as shown in Fig 14. A projection 41 is provided with a leg portion 41a projecting inward of the outer ring-shaped electrode 39, and a head portion 41b which is formed wider than the leg portion 41a, in the direction perpendicular to the projecting direction of the leg portions 41a.

A projection 41 penetrates through a hole 42 formed on the transparent tube 38, by being pushed from outside the transparent tube 38. A head portion 41b protrudes into the inner portion of the transparent tube 38, and the end portions of the hole 42 are put between the head portion 41b and an outer ring-shaped electrode 39 to fix the outer ring-shaped electrode 39 on the outer face of the transparent tube 38. An outer ring-shaped electrode 39 is configured to be connectable with a ring-shaped electrode 11 of the catheter 10 through this projection 41.

The outer side tip electrode 40 is in a state of a container which is a cylindrical body with an end closed, formed into the shape similar to the tip of the transparent tube 38, and comprises an electrode formed with a publicly-known material such as platinum alloys.

The outer side tip electrode 40 also has projections 41 as shown in Fig. 14 formed on plural points in the inner face thereof. The outer side tip electrode 40 is fixed to the tip of the transparent tube 38 through these projections 41. The outer side tip electrode 40 is configured to be connectable with the tip electrode 12 of the catheter 10, also through these projections 41.

The marker 45 comprises a publicly-known X-ray opaque marker, and is used to check whether a direction in which the light-emitting probe member 20 is positioned coincides with a direction to which the catheter 10 is bent. In a case that a direction in which the light-emitting probe member 20 is positioned is different from a direction to which the catheter 10 is bent, adjustment is made by rotating the catheter 10.

### <<Use of light-emitting probe P4 for a catheter >>

In the next place, a method of using the light-emitting probe P4 for a catheter of Fig. 12 by an operator who is a user will be described.

The light-emitting probe P4 for a catheter is provided in the state shown in Fig. 12.

An operator who is a user of the light-emitting probe P4 for a catheter disposes a selected catheter 10 into the space S in Fig. 12 so as to be fitted as in Fig. 13, to complete the laser catheter 4 with a light-emitting probe of Fig.11.

Although the light-emitting probe P4 for a catheter is provided in the stated shown in Fig. 12 in the present embodiment, it may also be provided in a state that the outer ring-shaped electrodes 39 and the outer side tip electrode 40 are not mounted to the transparent tube 38.

In this case, the operator may make the holes 42 by himself on the transparent tube 38 at positions coincided with the tip electrode 12 and the ring-shaped electrodes 11 of the catheter 10 selected by himself and engage the projections 41 with these holes 42, to thus mount the outer ring-shaped electrodes 39 and the outer side tip electrode 40.

### «Embodiment 8: Laser catheter 4 with a light-emitting probe»

The embodiment 7 described an example in which the laser catheter 4 with a light-emitting probe of Fig. 13 was produced by an operator who is a user by combining a light-emitting probe P4 for a catheter with a catheter 10 selected by himself. However, the present invention may be provided as in the embodiment of the laser catheter 4 with a light-emitting probe of Fig. 13 in a manner not limited thereto.

The laser catheter 4 with a light-emitting probe comprises a publicly known catheter 10 and the light-emitting probe member 20, fixed with each other by the cover 37, as shown in Fig. 13.

As for other configurations of the present embodiment, explanation is omitted, because they are similar to each configuration of the light-emitting probes P1 and P4 for a catheter of Embodiments 1 and 7.

### << Embodiment 9: Light-emitting probe P5 for a catheter >>

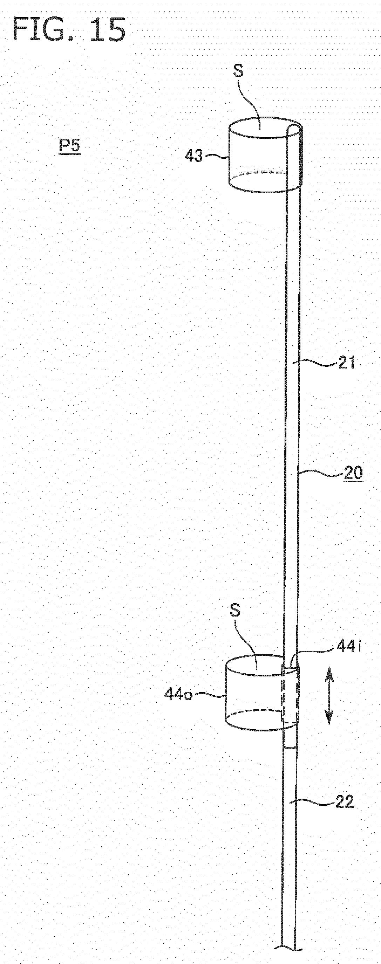

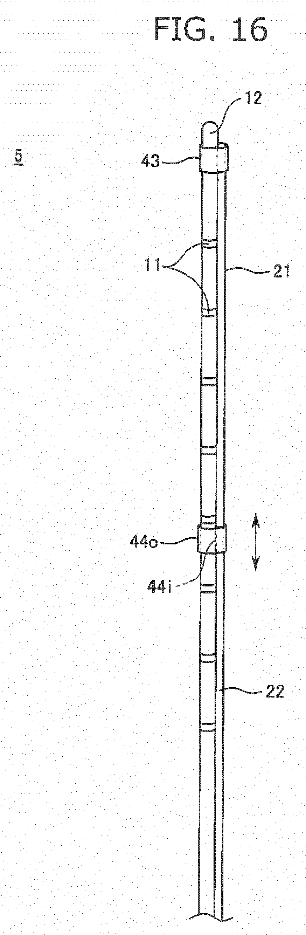

Still another example of the light-emitting probe for a catheter in the present invention will be described on the basis of Fig. 15 and Fig. 16.

The light-emitting probe P5 for a catheter of the present embodiment is provided with the light-emitting probe member 20 , a heat-shrinkable tube 43, in which the upper end of the light-emitting probe member 20 is welded to the inner face thereof; an inner tube 44i through which a portion in the vicinity of a boundary of a laser probe 21 and an optical fiber cable 22 of the light-emitting probe member 20 is inserted; and an outer tube 44o in which the inner tube 44i is welded to the inner face thereof, as shown in Fig. 15; and is provided as a kit for producing the laser catheter 5 with a light-emitting probe of Fig. 16.

The inner tube 44i is formed with a tube for medical use having a diameter a little larger than that of the light-emitting probe member 20. The heat-shrinkable tube 43 and the outer tube 44o are formed with a heat-shrinkable tube for medical use of a transparent fluoropolymer or the like, cut into a length which would become a length similar to the diameter of the catheter 10 after a heat shrinkage.

The outer tube 44o and the inner tube 44i are fixed with each other by welding, while the inner tube 44i and the light-emitting probe member 20 are not fixed with each other. Therefore, the inner tube 44i and the outer tube 44o are movable as one body in the direction of the arrow of Fig. 15 and Fig. 16 with respect to the light-emitting probe member 20.

The heat-shrinkable tube 43 and the outer tube 44o are provided with a space S inside, for the catheter 10 to be inserted adjacently to the light-emitting probe member 20 or the inner tube 44i.

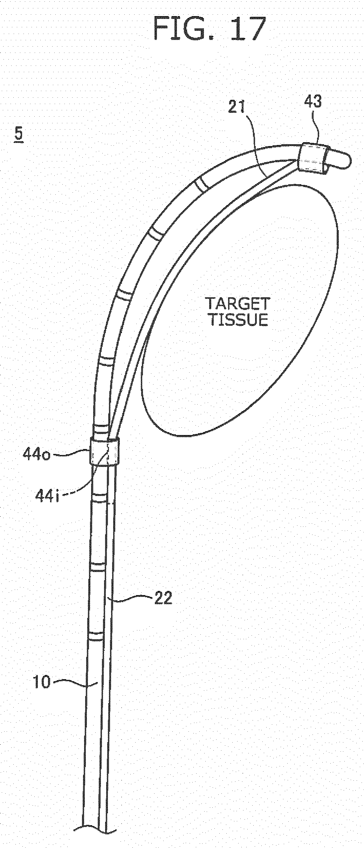

In the present embodiment, the light-emitting probe 20 is used with being curved, as shown in Fig. 17, when inserted into a living body. Therefore, for the curved shape to be able to be recognized under fluoroscopy by a fluoroscopic device, it is preferred that an opaque marker (not shown) is provided on the surface.

The opaque marker is formed with a publicly-known material for medical use, such as platinum, gold, iridium, and stainless steel; and is formed into any shape, such as a ring state and a coiled state.

It is preferred that the opaque marker is provided in plural numbers with being spaced from one another, so that a curved shape in a use of the light-emitting probe 20 can be recognized by the arrangement of the opaque markers arranged in plural numbers at a predetermined distance.

### <<Use of Light-emitting probe P5 for a catheter >>

In the next place, a method of using the light-emitting probe P5 for a catheter of Fig. 15 by an operator who is a user will be described.

Light-emitting probes P5 for a catheter is provided in the state shown in Fig. 15.

An operator who is a user of the light-emitting probe P5 for a catheter disposes a selected catheter 10 in the space S of Fig. 15, as in Fig. 16.

The heat-shrinkable tube 43 is disposed on a position avoiding the tip electrode 12 and the ring-shaped electrodes 11 of the catheter 10.

Then, the heat-shrinkable tube 43 and the outer tube 44o are shrunk by heating, and the catheter 10 is fixed inside the heat-shrinkable tube 43 and the outer tube 44o, to thus complete the laser catheter 5 with a light-emitting probe of Fig. 16.

Since the inner tube 44i and the light-emitting probe member 20 are not fixed to each other, the light-emitting probe P5 for a catheter can be switched between a position of Fig. 16 in which the catheter 10 and the light-emitting probe member 20 extend together to form substantially one body, and the position of Fig. 17 in which the catheter 10 is disposed in a longer distance than the length of the light-emitting probe member 20 between the heat-shrinkable tube 43 and the outer tube 44o, so that the light-emitting probe member 20 is made into a shape like a bow string, by operating a control handle at hand (not shown) by an operator.

Accordingly, the laser catheter 5 with a light-emitting probe is switched to the arrangement of Fig. 16 when moved forward in a blood vessel, and switched to the arrangement as in Fig. 17 when a treatment of a target tissue is performed, so that the light-emitting probe P5 for a catheter is pressed against the target tissue, which facilitate bringing the laser probe 21 into close contact with the target tissue.

Incidentally, the light-emitting probe P5 for a catheter of the present embodiment comprises a two-layered structure of the inner tube 44i and the outer tube 44o. However, the structure is not limited thereto, but it is only necessary that the tip of the light-emitting probe member 20 and the tip side of the catheter 10 are fixed to each other, and light-emitting probe member 20 and the catheter 10 are bundled movably with respect to each other in the longitudinal direction at a position in the vicinity of the boundary of the laser probe 21 and the optical fiber cable 22.

For example, instead of the two-layered structure of the inner tube 44i and the outer tube 44o, the light-emitting probe member 20 and the catheter 10 may be bundled by a tube of single layered structure which does not fix the light-emitting probe member 20 and the catheter 10 to each other. It is preferred that this tube is constructed with a tube other than heat-shrinkable tubes, a light-emitting probe member 20 is fixed to the inner face thereof at a position in the vicinity of the boundary of the laser probe 21 and the optical fiber cable 22, and the catheter 10 is inserted therein without being fixed.

### <<Embodiment 10: Laser catheter 5 with a light-emitting probe>>

Embodiment 9 described an example in which the laser catheter 5 with a light-emitting probe of Fig. 16 or Fig. 17 was produced by an operator who is a user, by combining a light-emitting probe P5 for a catheter with a catheter 10 selected by himself. However, the present invention may be provided as in the embodiment of the laser catheter 5 with a light-emitting probe of Fig. 16 or Fig. 17 in a manner not limited thereto.

As for other configurations of the present embodiment, explanation is omitted, because they are similar to each configuration of the light-emitting probes P1 and P5 for a catheter of Embodiments 1 and 9.

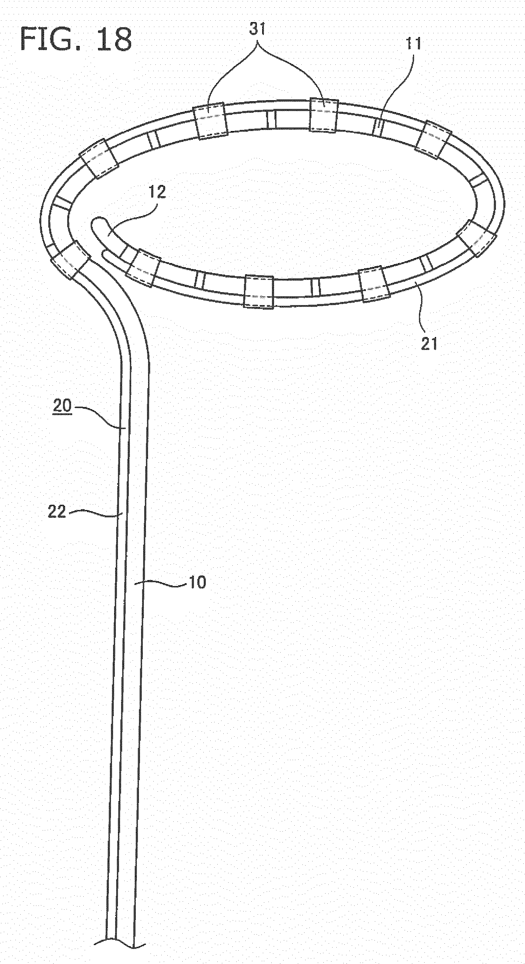

Incidentally, the each embodiment above showed a catheter 10 in which the tip side could be curved as in Fig. 1. However, it is also possible to use a catheter 10 in which the tip side of the catheter 10 is formed into a ring state such that the tip side of the catheter 10 closely faces or contacts with another portion of the catheter 10, as in Fig. 18.

In addition, in the each embodiment above, in order to exploit the functions of the ring-shaped electrodes 11 and the tip electrode 12 of the catheter 10, the catheter 10 is partially exposed from an externally mounted member, or the cover 37 is provided with a conductive body to electrically connect the ring-shaped electrodes 11 and the tip electrode 12 with an outer portion of the cover 37, as in Embodiments 2, 4, 6, 8, and 10 shown in Fig. 5, Fig. 8, Fig. 11, Fig. 13, Fig. 16, and Fig. 17. However the functions of the ring-shaped electrodes 11 and the tip electrode 12 may be maintained by other method not limited to thereto.

For example, instead of providing the outer ring-shaped electrodes 39 and the outer side tip electrode 40 to the cover 37 of Fig. 13, it is also possible that the entire surface of the cover 37 is formed with an anisotropic conductive body having a conductivity in the thickness direction of the cover 37, and an insulation in directions different from the thickness direction.

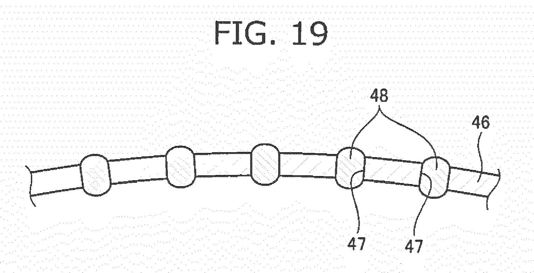

The anisotropic conductive body is constructed, for example, with an insulating film 46 having an electrical insulation property, and conductive bumps 48 that are filled in plural fine through-holes 47 formed on the insulating film 46, as shown in Fig 19.

The insulating film 46 is constructed with a thermosetting resin or a thermoplastic resin which is preferably used in medical use, and the conductive bumps 48 are constructed with various metals such as gold, silver, tin or the like, or various alloys comprising various metals, or the like.

It is also possible to use a heat-shrinkable tube comprising the anisotropic conductive body having a conductivity in the thickness direction and an insulation in directions different from the thickness direction, instead of the mesh tube 36 shown in Fig. 10 and Fig. 11.

### << Embodiment 11: Light-emitting probe for an endoscope>>

The light-emitting probes P1 to P5 of the each embodiment above can also be a light-emitting probe for an endoscope, by using an endoscope in stead of the catheter 10.

The light-emitting probe for an endoscope of the present embodiment can preferably be used in a photodynamic therapy performed under an endoscope for pancreatic cancer, biliary tract cancer or the like, in which a very thin small-diameter endoscope, for example, a biliary tract endoscope (outer diameter 1 to 3 mm) or a pancreas endoscope (outer diameter 1 to 2.5 mm) is used.

A pancreatic cancer or a biliary tract cancer is present in periphery of thin tracts such as a pancreatic duct or biliary tract. Therefore, an endoscope having an ordinary thickness equipped with an adequate treatment tools, etc. inside can not reach the lesion portion. In contrast, if a light-emitting probe 20 can be externally mounted to a very thin biliary tract endoscope 13 as in the present embodiment, overall thickness of an obtained endoscope with a light-emitting probe is still kept thin, which allows a biliary tract endoscope 13 to be moved forward to the lesion portion, and a photodynamic therapy to be performed with using the light-emitting probe 20.

The endoscope used in the present embodiment preferably is a small-diameter endoscope, such as pancreatic duct mirrors, biliary tract endoscopes (outer diameter 1 to 3 mm), pancreas endoscopes (1 to 2.5 mm), nasal endoscopes (external diameter 5 mm), etc.

The biliary tract endoscope may be, for example, a child scope of a parent-child scope in which a parent scope accommodates a child scope. In the case of parent-child scope, for example, the parent-child scope is orally moved forward to duodenum, then a papilla port and a bile duct port are dissected from duodenal papilla by the parent scope (duodenum endoscope), and a child scope (biliary tract endoscope) projected to the outer side of the parent scope is moved forward to the bile duct, to carry out an observation. In the case of pancreatic duct mirror, a papilla port and a bile duct port are dissected by the parent scope (endoscopic papillotomy), and a pancreatic duct mirror (child scope) is inserted into the pancreatic duct to carry out an observation of intraductal papillary tumors and pancreatic cancer.

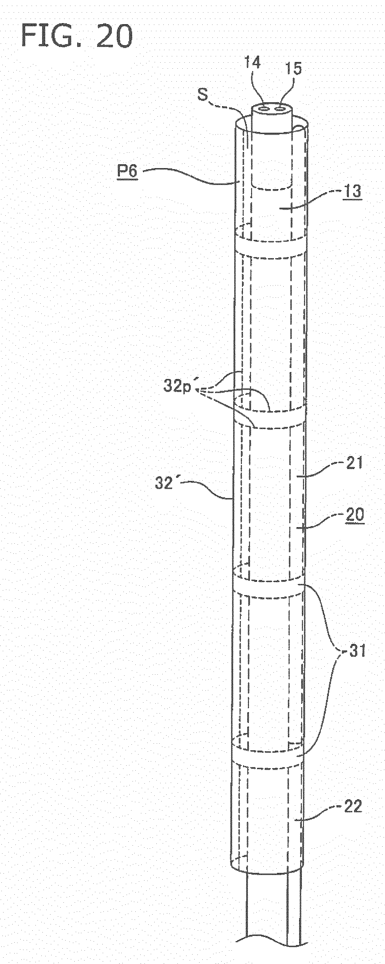

Fig 20 shows an example of applying a light-emitting probe P6 for an endoscope having the same structure with the light-emitting probe P1' for a catheter of Fig. 6 to a biliary tract endoscope 13.

As shown in Fig. 20, the light-emitting probe P6 for an endoscope of the present embodiment is provided with a light-emitting probe member 20 and a heat-shrinkable tube 32' having the light-emitting probe member 20 inserted therein, and is provided as a kit for producing an endoscope 6 with a light-emitting probe.

On the side face of the heat-shrinkable tube 32', thin portions 32p' which have a similar pattern with that of the light-emitting probe P1' for a catheter are formed, and on the inner face of the heat-shrinkable tube 32', a light-emitting probe member 20 is firmly fixed along the longitudinal direction.

The biliary tract endoscope 13 used in the present embodiment is capable of being inserted into a pancreatic duct or a biliary tract and is composed of a very thin typical small-diameter endoscope with an outer diameter of 5.9 mm or less, for example, 1 to 3 mm, and provided inside with an objective optical system 14 for observing a designated area and an illumination optical system 15 capable of irradiating the designated area observable by the objective optical system.

Here, the biliary tract endoscope 13 of the present embodiment may be provided with a very thin instrument inside and may be provided with an electrode on the outer face.

The light-emitting probe P6 for an endoscope is provided with a space S in a position adjacent to the light-emitting probe member 20 in the heat-shrinkable tube 32'. An operator who is a user inserts a biliary tract endoscope 13 into the space S as shown in Fig. 20, and shrink the heat-shrinkable tube 32' by heating, subsequently, tears the heat-shrinkable tube 32' along the thin portions 32p' with leaving the ring tubes 31', to thus produce the endoscope with a light-emitting probe (not shown).

Incidentally, the present invention may also be provided as an embodiment of an endoscope with a light-emitting probe, not as in the embodiment of the light-emitting probe P6 for endoscope. It is also possible that the light-emitting probes P1 to P5 of the each embodiment described above may be externally mounted to an endoscope, instead of the catheter 10.

Explanations for other configurations of the light-emitting probe P6 for an endoscope and the endoscope with a light-emitting probe produced with the light-emitting probe P6 for an endoscope and a biliary tract endoscope 13 are omitted because they are similar to those in Fig. 6 and Fig. 7.

Incidentally, while normally a catheter 10 is used only once, a biliary tract endoscope 13 is not discarded after use, but used repeatedly. Therefore, in order to facilitate separating a biliary tract endoscope 13 after use, the ring tube 31' may be provided with thin portions (not shown) formed to be more vulnerable than other portions of the ring tube 31' although a little thicker than the thin portions 32p', or a notch, an opening or the like which facilitate a tearing from the end portion.

The light-emitting probe P6 for an endoscope of Fig. 6 may be applied not only to a biliary tract endoscope 13 but also to other endoscopes, and may also be applied to other medical devices such as sheaths.

The present invention may also be realized in the following structure, in addition to the structures recited inembodiments.

That is, the heat-shrinkable tube may be provided with plural ring-shaped heat-shrinkable tubes disposed on plural points in the longitudinal direction of a light-emitting probe with being spaced from one another, and a stripping tube fixed on the external faces of the plural ring-shaped heat-shrinkable tubes so as to be removable after a heat shrinkage.

Being thus configured allows producing a medical device with a light-emitting probe only by inserting a medical device into the space formed with the ring-shaped heat-shrinkable tubes and the stripping tube, then shrinking these tubes by heating, and thereafter, stripping the stripping tube from the vulnerable portions, and thus, an operator can easily produce a medical device with a light-emitting probe in a simple operation.

Moreover, in the produced medical device with a light-emitting probe, since the periphery of the medical device and the light-emitting probe is provided only with the ring-shaped heat-shrinkable tubes, operation of the medical device is not inhibited.

It is also possible that the heat-shrinkable tube comprises a single layer and part of the heat-shrinkable tube is partially removable after a heat shrinkage.

For being thus configured, even in a case that a medical device is provided with an electrode on the outer periphery thereof, the electrode is exposed on an outer side of a heat-shrinkable tube, to cause no hindrance in measuring an electric potential.

It is also possible that a tube for light-emitting probe which extends along the extending direction of the heat-shrinkable tube is fixed to the side face of the heat-shrinkable tube, and the light-emitting probe is fixed inside the tube for light-emitting probe.

In addition, the heat-shrinkable tube may be formed with a mesh.

For being thus configured, it is possible to take a potential from between the mesh of a heat-shrinkable tube, and thus it is possible to preferably use the device also in a medical device comprising an electrode such as an electrode catheter.

The externally mounting section comprises a bag-shaped body provided with a bag-shaped space which is capable of accommodating the tip of the light-emitting probe and capable of accommodating the tip of the medical device. The bag-shaped body is provided with an external electrode on the external face. The external electrode may comprise a connecting portion which penetrates the bag-shaped body in the thickness direction so as to be connectable to an electrode of the medical device accommodated inside the bag-shaped body.

Since the external face is provided with an external electrode which comprises a connecting portion penetrating the bag-shaped body in the thickness direction so as to be connectable to an electrode of the medical device accommodated inside the bag-shaped body, it is possible to take a potential from outside the bag-shaped body, and therefore, the device can preferably used also in a medical device which is provided with an electrode, such as an electrode catheter.

It is also possible to provide a pair of the heat-shrinkable tubes, with one of the heat-shrinkable tubes fixed to the tip side of the light-emitting probe, while the other of the heat-shrinkable tubes holding the end side in the opposite side of the tip of the light-emitting probe such that the light-emitting probe is movable in the longitudinal direction with respect to the other heat-shrinkable tube. The one of the heat-shrinkable tubes is capable of being externally mounted to the tip side of the medical device, while the other heat-shrinkable tube may be capable of being externally mounted to a position separated from the tip side of the medical device by a distance longer than the length to the end side in the opposite side of the tip. Since the one of the heat-shrinkable tubes is capable of being externally mounted to the tip side of the medical device, while the other heat-shrinkable tube is capable of being externally mounted to a position separated from the tip side of the medical device by a distance longer than the length to the end side in the opposite side of the tip in this manner, it becomes possible, when a medical device with a light-emitting probe is moved forward in a narrow region such as a vascular, that the medical device and the light-emitting probe are passed through the region along each other, and when reached to a target tissue for treatment, the light-emitting probe is drawn by a control handle at hand, by which the medical device is curved like a bow and the light-emitting probe becomes like a string, to thus facilitate pressing the light-emitting probe against the target tissue for treatment.

In each embodiment of the present specification, the medical device and the light-emitting probe are connected to each other by a tube or a cover of resin or the like. However, without limitation, the medical device and the light-emitting probe may also be connected to each other, for example, by a coil or a C-ring which comprises a shape memory alloy. In this case, the coil or the C-ring may be made discontinuous in the longitudinal direction of the medical device and provided so as to be superimposed around an electrode of the medical device.

### Reference Numerals

P1, P1', P2, P3, P4, P5: Light-emitting probe for a catheter
P6: Light-emitting probe for an endoscope
S: space
1, 2, 3, 4, 5: Laser catheter with a light-emitting probe
6: Endoscope with a light-emitting probe
10: Catheter
11: Ring-shaped electrode
12: Tip electrode
13: Biliary tract endoscope
14: Objective optical system
15: Illumination optical system
20: Light-emitting probe member
21: Laser probe
22: Optical fiber cable
23: Core
24: Cladding
25: Covering
26: Tip portion
27: Resin layer
28: Light diffusing body
31, 31': Ring tube
32: Stripping tube
32p, 32p', 35p, 35p': Thin portion
32', 43, 44: Heat-shrinkable tube
33: Double tube
34: First tube
35: Second tube
35a, 35a': Region to be stripped off
35b, 35b': Ring portion
35c, 35'c: Connecting portion
36: Mesh tube
37: Cover
38: Transparent tube
39: Outer side ring-shaped electrode
44: Outer side tip electrode
41: Projection
41a: Leg portion
41b: Head portion
42: Hole
44i: Inner tube
44o: Outer tube
45: Marker
46: Insulating film
47: Fine through-holes
48: Conductive bump

## Claims

1. A kit for mounting a light-emitting probe for a medical device, comprising:
a light transmission section that transmits a light from a light source;
a light-emitting probe that is connected to the light transmission section and emits a light transmitted by the light transmission section; and
an externally mounting section for externally mounting the light-emitting probe to an outer portion of a medical device used by being inserted into a living body.

2. The kit for mounting a light-emitting probe for a medical device according to claim 1, wherein a light emitting face of the light-emitting probe is provided on a side face of the light-emitting probe, and continuously extended along an extending direction of the light-emitting probe.

3. The kit for mounting a light-emitting probe for a medical device according to claim 1 or 2, wherein the externally mounting section comprises a heat-shrinkable tube comprising a space which is capable of being inserted with the medical device inside prior to a heat shrinkage, the heat-shrinkable tube being fixed to the light-emitting probe.

4. The kit for mounting a light-emitting probe for a medical device according to any one of claims 1 to 3, wherein the externally mounting section is capable of externally mounting plural number of the light-emitting probes to a single body of the medical device.

5. The kit for mounting a light-emitting probe for a medical device according to any one of claims 1 to 4, wherein the externally mounting section comprises an opening that exposes an electrode formed on an external face of the medical device.

6. The kit for mounting a light-emitting probe for a medical device according to any one of claims 1 to 5, wherein the externally mounting section comprises a covering portion that covers a surface of the medical device, the covering portion comprising an anisotropic conductive body having a conductivity in the thickness direction, and having an insulation in a direction different from the thickness direction.

7. The kit for mounting a light-emitting probe for a medical device according to any one of claims 1 to 6, wherein the medical device is a medical device selected from the group consisting of catheters, sheaths and endoscopes.

8. A medical device, comprising
a light transmission section that transmits a light from a light source,
a light-emitting probe that is connected to the light transmission section and emits a light transmitted by the light transmission section,
a medical device that is used by being inserted into a living body, and
an externally mounting section for externally mounting the light-emitting probe to an outer portion of the medical device.
